# EUROPEAN PATENT APPLICATION

(11) **EP 4 393 432 A2**
(43) Date of publication of application: **03.07.2024**
(21) Application number: 23219832.5
(22) Date of filing: 22.12.2023
(51) Int. Cl.: A61B 18/14

(54) **ABLATION CATHETER WITH EXPANDABLE WOVEN MESH HAVING ELECTRICALLY CONDUCTIVE STRANDS**

(30) Priority: 29.12.2022 US 202263477664 P; 13.11.2023 US 202318507411
(71) Applicant: BIOSENSE WEBSTER (ISRAEL) LTD., 2066717 Yokneam (IL)
(72) Inventor: BEECKLER, Christopher Thomas, Irvine, 92618 (US); KEYES, Joseph Thomas, Irvine, 92618 (US); OKARSKI, Kevin Mark, Irvine, 92618 (US); JENKINS, Nathaniel, Irvine, 92618 (US); HERRERA, Kevin Justin, Irvine, 92618 (US); KHAN, Ishan, Irvine, 92618 (US)
(74) Representative: Carpmaels & Ransford LLP

(57) **Abstract**

The disclosed technology includes an end effector in a medical catheter including a woven mesh extending along a longitudinal axis from a proximal portion to a distal portion, the woven mesh defining a generally spheroidal shape having a plurality of electrodes disposed about the longitudinal axis and adjacent to an equator of the generally spheroidal shape with the proximal portion configured to be attached to a shaft and with the distal portion configured to be invaginated and coupled to an actuator extending along the longitudinal axis.

## Description

### CROSS-REFERENCE TO RELATED APPLICATIONS

This application claims the benefit of prior filed U.S. Provisional Patent Application No. 63/477,664 filed on December 29, 2022, which is hereby incorporated by reference as set forth in full herein.

### FIELD

The present invention relates generally to medical devices, and in particular medical probes with electrodes, and further relates to, but not exclusively, medical probes suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. By selectively ablating cardiac tissue by application of energy via an electrode catheter, it is sometimes possible to cease or modify the propagation of unwanted electrical signals from one portion of the heart to another.

In use, the electrode catheter is inserted into a major vein or artery, e.g., femoral vein, and then guided into the chamber of the heart which is of concern.

Catheters with flexible tips are known. U.S. Pat. No. 5,720,719 describes a catheter having a probe end that includes a malleable tube and a flexible tube. The medical probe may include means for inflating the deformable end which may include conveying a fluid that irrigates the tissue through pores of the deformable distal end. The means for inflating the deformable distal end may include conveying the fluid so as to generate a mechanical force sufficient to inflate the deformable distal end. A contact area between the deformable distal and the tissue can increase upon pressing the deformable distal end against the tissue.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Some ablation approaches use irreversible electroporation (IRE) to ablate cardiac tissue using nonthermal ablation methods. IRE delivers short pulses of high voltage to tissues and generates an unrecoverable permeabilization of cell membranes. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached to the Appendix of priority patent Application No. 63/477,664.

Regions of cardiac tissue can be mapped by a catheter to identify the abnormal electrical signals. The same or different medical probe can be used to perform ablation. What is needed, therefore, are multifunction atraumatic end effectors for electrode catheters.

### SUMMARY

There is provided, in accordance with an embodiment of the present invention, an end effector in a medical catheter including a woven mesh extending along a longitudinal axis from a proximal portion to a distal portion, the woven mesh defining a generally spheroidal shape having a plurality of electrodes disposed about the longitudinal axis and adjacent to an equator of the generally spheroidal shape with the proximal portion configured to be attached to a shaft and with the distal portion configured to be invaginated and coupled to an actuator extending along the longitudinal axis.

The disclosed technology can further include an electrode including a woven mesh having a collapsed configuration and a radially expanded deployed configuration, and a plurality of exposed electrically conductive strands configured to deliver ablation energy to tissue and providing a cumulative contact area of about 0.004 square inches or greater to tissue such that the cumulative contact area is a sum total of contact area of exposed regions of the plurality of exposed electrically conductive strands to tissue to deliver at least 900 volts and a current of about 20 amperes.

The disclosed technology can further include a medical probe including a woven mesh having a plurality of electrically conductive strands configured to deliver ablation energy to tissue and a plurality of sensors coupled to the woven mesh.

The disclosed technology can further include an atraumatic medical probe including a woven sheet having a first plurality of wires interwoven with a second plurality of wires and formed into a teardrop shape with a blunt distal end and a tapered proximal end, with at least a portion of the second plurality of wires being configured to deliver ablation energy to tissue, and a sensor attached to at least one of the first plurality of wires.

The disclosed technology can further include a method of making an atraumatic medical probe, the method including forming a flat woven sheet having a first plurality of wires and a second plurality of wires into a teardrop shape having a blunt distal end and a tapered proximal end, configuring the second plurality of wires to deliver ablation energy to tissue, and attaching a sensor to at least one of the first plurality of wires.

The disclosed technology can further include a basket for a medical probe including a woven sheet having a first plurality of wires oriented in a first direction, each of the first plurality of wires having a first end and a second end, and a second plurality of wires oriented in a second direction parallel to the first direction, the first plurality of wires and the second plurality of wires woven together in a plain weave pattern and formed into a teardrop shape having a blunt distal end and a tapered proximal end, each of the second plurality of wires having a first end and a second end, the second plurality of wires interwoven with the first plurality of wires, the first end and the second end of at least one of the first plurality of wires joined to another of the first plurality of wires, and the first end and the second end of at least one of the second plurality of wires joined to another of the second plurality of wires.

### BRIEF DESCRIPTION OF THE DRAWINGS

FIG. 1 is a schematic pictorial illustration of a medical system including a medical catheter with a distal end that includes an end effector having a woven mesh, in accordance with an embodiment of the present invention;
FIG. 2A is a schematic pictorial illustration showing a perspective view of an end effector of a medical catheter, in accordance with an embodiment of the present invention;
FIG. 2B is a schematic pictorial illustration showing a perspective view of an end effector of a medical catheter including a pusher rod, in accordance with an embodiment of the present invention;
FIG. 3 is a schematic pictorial illustration showing a side view of an end effector in a collapsed configuration, in accordance with the disclosed technology;
FIG. 4 is a schematic pictorial illustration showing a side view of a distal end of a catheter shaft joining an end effector in a collapsed configuration, in accordance with the disclosed technology;
FIG. 5 is a schematic pictorial illustration showing a sensor or electrode coupled to a woven mesh and viewed from an internal volume of the braided mesh, in accordance with the disclosed technology;
FIG. 6 is a schematic pictorial illustration showing a side view of a sensor or electrode coupled to a woven mesh of FIG. 5, in accordance with the disclosed technology;
FIG. 7 is a schematic pictorial illustration showing a perspective view of an atraumatic medical probe, in accordance with the disclosed technology;
FIG. 8 is a schematic pictorial illustration showing a side view of an atraumatic medical probe, in accordance with the disclosed technology;
FIG. 9 is a schematic pictorial illustration showing a top view of a woven mesh, in accordance with the disclosed technology;
FIG. 10A is a schematic pictorial illustration showing a perspective view of a woven mesh, in accordance with the disclosed technology;
FIG. 10B is a schematic pictorial illustration showing a perspective view of a woven mesh, in accordance with the disclosed technology;
FIG. 11 is a schematic pictorial illustration showing a side view of a woven mesh, in accordance with the disclosed technology;
FIG. 12A is a schematic pictorial illustration showing a side view of a woven mesh, in accordance with the disclosed technology;
FIG. 12B is a schematic pictorial illustration showing a magnified view of the woven mesh of 12A, in accordance with the disclosed technology;
FIG. 13 is a schematic pictorial illustration showing a perspective view of a basket, in accordance with the disclosed technology;
FIG. 14 is a schematic pictorial illustration showing a perspective view of an electrode, in accordance with the disclosed technology;
FIG. 15 is a schematic pictorial illustration showing a perspective view of an electrode, in accordance with the disclosed technology; and
FIG. 16 is a flowchart illustrating a method of making an atraumatic medical probe, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference and attached to the Appendix of priority patent Application No. 63/477,664.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference and attached to the Appendix of priority patent Application No. 63/477,664.

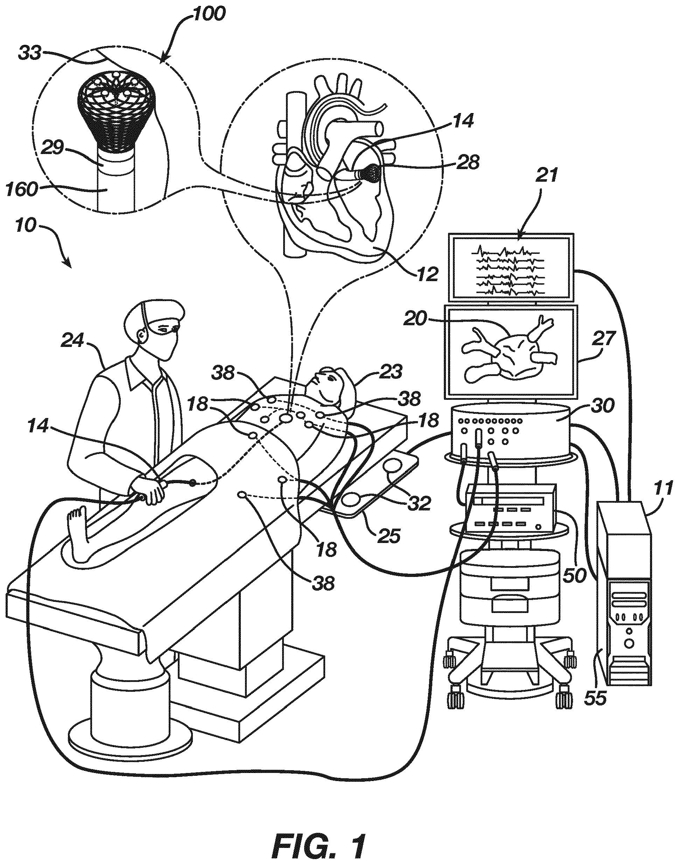

FIG. 1 illustrates an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example medical probe 14 is illustrated herein. As used herein, the term "medical probe" includes a catheter or medical catheter. Physician 24 brings an end effector 100 at a distal tip of medical probe 14 into contact with the heart wall 33 for sensing and/or ablating a target site in heart 12.

The end effector 100 can include one or more electrodes for sensing and/or ablating tissue. The end effector 100 can further include one or more electrodes which can function as impedance based position sensors.

Medical probe 14 may additionally include a position sensor 29 in the shaft of the medical probe 14 near the end effector 100 for tracking position and orientation of end effector 100. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of the end effector 100 of medical probe 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes (positioned in end effector 100 and/or shaft). For impedance-based tracking, electrical current is directed toward electrodes near the distal end of the medical probe 14 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and/or intracardiac electrograms (IEGM) captured with electrodes of medical probe 14. Recorder 11 may include pacing capability for pacing the heart rhythm and/or may be electrically connected to a standalone pacer.

System 10 may include an ablation energy generator 50 that is adapted to conduct ablative energy to one or more of electrodes of the end effector 100 of the medical probe 14. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between medical probes (catheters), electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the medical probes and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

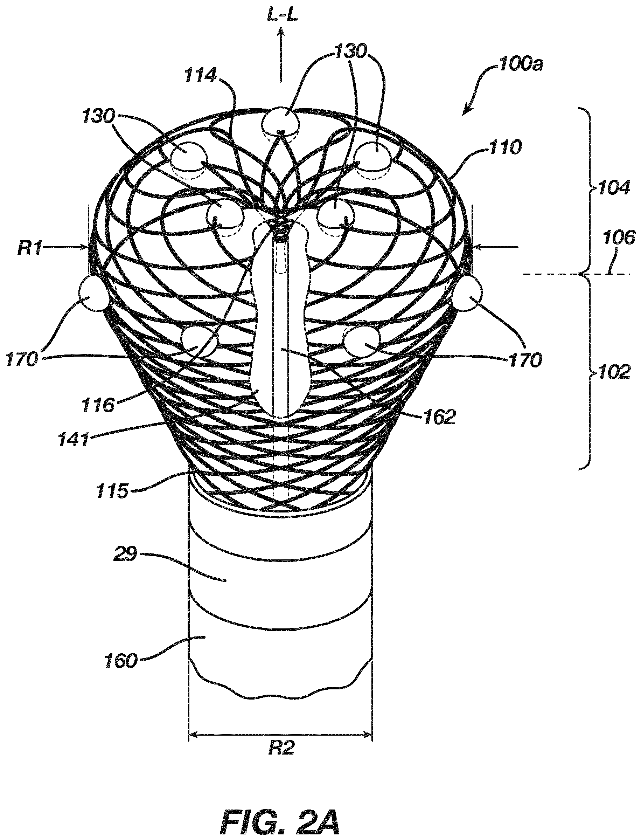

FIG. 2A shows an end effector 100a including a woven mesh 110 extending along a longitudinal axis L-L from a proximal portion 102 to a distal portion 104. The woven mesh 110 can define a generally spheroidal shape having a plurality of electrodes 170 disposed about the longitudinal axis L-L and adjacent to an equator 106 of the generally spheroidal shape. The electrodes 170 can include gold, platinum, and palladium (and their respective alloys). These materials also have high thermal conductivity which allows the minimal heat generated in the tissue (i.e., by the ablation energy delivered to the tissue) to be conducted through the electrodes to the back side of the electrodes (i.e., the portions of the electrodes on the inner sides of the spines), and then to the blood pool in heart 12.

The proximal portion 102 can be configured to be attached to a shaft 160. The distal portion 104 can be configured to be invaginated and coupled to an actuator 162 extending along the longitudinal axis L-L, meaning the woven mesh 110 is folded into itself at a distal end 114 of the woven mesh 110 to form an inner braid portion 116 that is attached to the actuator 162. The actuator 162 is positioned inside of a sack formed by the woven mesh 110 and defining an inner volume of the woven mesh. The actuator 162 can serve to transition the device from a collapsed configuration and a radially expanded deployed configuration.

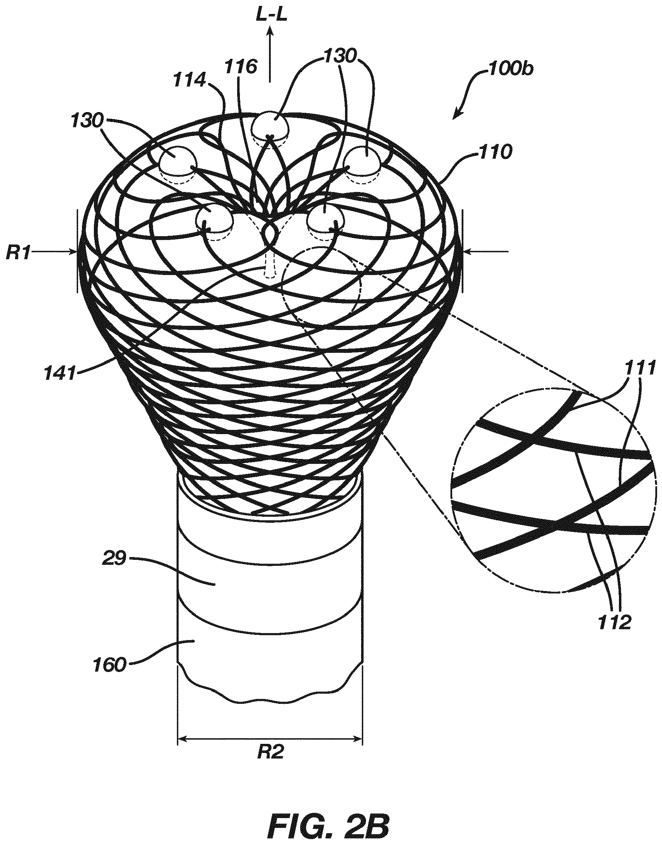

FIG. 2B shows a modified end effector 100b of a medical catheter 14 that is similar to the end effector 100a illustrated in FIG. 2A but with the actuator 162 and the electrodes 170 at the equator 106 removed. The woven mesh 110 can be configured to self-expand to form the generally spheroid shape, and can include a resilient material and/or a memory shape material such as nickel titanium alloy (nitinol). The woven mesh 110 includes a distal portion 104 that can be invaginated to form an atraumatic distal end 114 and a proximal portion 102 configured to be attached to a shaft 160 as indicated in FIG. 2A.

Referring collectively to FIGs. 2A and 2B to illustrate features of the end effector 100 of FIG. 1, the end effector 100 can include an actuator as illustrated in FIG. 2A or the woven mesh 110 can be configured to self-expand without the aid of an actuator as illustrated in FIG. 2B.

The end effector 100 can include one or more electrode(s) 170 that are attached to the woven mesh 110 and structurally supported by the woven mesh 110. The electrodes 170 can be positioned at any desirable location on the woven mesh 110, preferably at the equator 106 as illustrated in FIG. 2A or at the distal end 114 (e.g. in place of sensors 130). Electrodes 170 can function for sensing IEGM signals, impedance-based navigation, and/or ablation as disclosed in relation to FIG. 1 and as otherwise understood by a person skilled in the art. As an alternative to having electrodes 170 attached to the woven mesh 110, the woven mesh itself can include one or more conductive strands with one or more exposed portions configured to contact tissue and function as an electrode for sensing IEGM signals, impedance-based navigation, and/or ablation as disclosed in relation to FIG. 1 and as otherwise understood by a person skilled in the art.

The end effector 100 can include one or more sensor(s) 130 that are attached to the woven mesh 110 and structurally supported by the woven mesh 110. The sensors 130 can be positioned at any desired location on the woven mesh 110, preferably at the distal end 114, or at the equator 106 (e.g. in place of electrodes 170 in FIG. 2A). Sensors 130 can function to determine deformation of the woven mesh, sense temperature, measure electrical activity of tissue (e.g. IEGM signals), and/or perform other functions of an end effector sensor as understood by a person skilled in the art.

The woven mesh 110 can include electrically conductive strands facilitate electrical connection of the electrodes 170 and/or sensors 130 to the PIU 30 of system 10 (FIG. 1). The conductive strands can be attached to wires, or other conductors in the shaft 160 of the medical probe 14 and/or can extend through the shaft 160 to a connector at a proximal end of the medical probe 14. Additionally, or alternatively, the end effector 100a can include conductors (e.g. wires) electrically connected to the electrodes 170 and/or sensors 130 that are positioned within the sack of the woven mesh 110 and not woven into the woven mesh 110 such that the conductors provide electrical connection of the electrodes 170 and/or sensors 130 to the PIU of system 10 (FIG. 1). Strands 111, 112 (FIG. 2B) of the woven mesh 110 can be insulated and/or have exposed electrically conductive portions.

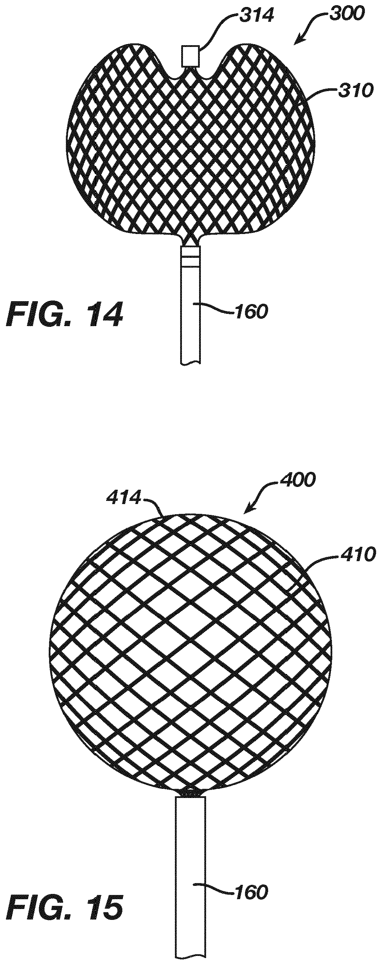

As illustrated FIG. 2B, the woven mesh 110 of the end effector 100 (FIG. 1) can include first strands 111 in a first direction and second strands 112 in a second direction. The woven mesh can be formed from a woven sheet or a braided tube that is shaped to the generally spherical shape. The first strands 111 run next to each other and do not overlap within end effector 100, and the second strands 112 run next to each other and do not overlap within the end effector 100. The woven mesh 110 extends along the longitudinal axis L-L distally from a distal end of the shaft 160. The woven mesh 110 includes weave ends disposed within a distal portion of the shaft 160. The woven mesh 110 can include weave ends 141 (FIG. 2B) in the inner braid portion 116 near the distal end 114 of the medical probe 14 or can have a contiguous weave distal of the distal end of the shaft (see FIG. 15). The weave ends of the inner braid portion 116 can be positioned within an internal volume defined by the woven mesh 110.

In one example, the woven mesh 110 can include electrically insulated strands and exposed electrically conductive strands. The plurality of electrically insulated strands can run along each other and overlap the exposed electrically conductive strands. For instance, at least some of the first strands 111 can be electrically insulated strands, and at least some of the second strands 112 can be exposed electrically conductive strands. The electrically insulated strands can be electrically conductive strands with an outer insulation. The electrically insulated strands can be connected to electrodes 170 and/or sensors 130 to provide connection from the electrodes 170 and/or sensors 130 to the PIU 30 (FIG. 1). The exposed electrically conductive strands can be configured to electrically connect to the PIU 30 so that the exposed electrically conductive strands can function as one or more electrodes of the end effector 100 to provide IEGM signal sensing, impedance-based navigation, and/or ablation as disclosed in relation to FIG. 1 and as otherwise understood by a person skilled in the art. The exposed electrically conductive strands can include a biocompatible memory shape metal such as a nickel titanium alloy (e.g. nitinol) or other such metal as understood by a person skilled in the art with suitable memory shape and electrical conductivity properties. The exposed electrically conductive strands can each have a thickness of approximately 25 micrometers or greater.

Strands 111, 112 can be selectively electrically connected together ("shorted") within the shaft 160, at the inner braid portion 116 and/or in the shaft 160 of the catheter 14 in various combinations as understood by a person skilled in the art. The exposed electrically conductive strands can be individually insulated from each other to function as individual electrodes or at least a portion of the exposed electrically conductive strands can be electrically connected together so that multiple exposed electrically conductive strands function as a single electrode. One or more exposed electrically conductive strands that form one electrode can be configured to provide a cumulative contact area of about 0.004 square inches or greater to tissue, or about 0.006 square inches or greater to tissue such that the cumulative contact area is a sum total of contact area of exposed region(s) of the exposed electrically conductive strand(s) of the electrode to tissue.

In one example, a majority or all of the first strands 111 and the second strands 112 are exposed electrically conductive strands and are shorted to form a single electrode configured to deliver ablation energy to tissue (e.g. IRE or RF). The exposed electrically conductive strands can each have a thickness of approximately 25 micrometers or greater. The sum total of contact area of exposed region(s) of the exposed electrically conductive strands of the single electrode to tissue provides a cumulative contact area of about 0.004 square inches or greater to tissue, or about 0.006 square inches or greater to tissue. The exposed electrically conductive strands of the single electrode can be configured to delivery at least 900 volts and a current of about 20 amperes.

The end effector 100 includes one or more sensors 130 coupled to the woven mesh 110 and electrically insulated from the first strands 111 and the second strands 112 of the woven mesh 110. The end effector 100 includes one or more wires electrically insulated from the first strands 111 and the second strands 112 and electrically coupled to the sensor(s) 130. The wire(s) coupled to the sensor(s) provide an electrical connection to the PIU 30, and the sensor(s) are configured to determine deformation of the woven mesh, sense temperature, and/or measure electrical activity of tissue. At least some of the wire(s) coupled to the sensor(s) can be routed within the internal volume of the woven mesh 110 without interleaving with the mesh 110, and/or at least some of the wire(s) coupled to the sensor(s) can be woven into the mesh either in place of a strand 111, 112, in line with a strand 111, 112, or interlaced inconsistent with the weave pattern.

In one example, the end effector 100 can include one or more electrodes that include an electrode 130 coupled to the woven mesh 110 and an exposed electrically conductive strand. The electrode 130 and the exposed electrically conductive strand can be shorted together to form a single electrode or can be insulated from each other to form two distinct electrodes.

In one example, at least some of the first strands 111 are electrically insulated strands that are connected to sensors 130 and electrically insulated from the second strands 112 which are exposed electrically conductive strands. The second strands 112 are shorted to form a single electrode configured to deliver ablation energy to tissue (e.g. IRE or RF). The sensors 130 are coupled to the woven mesh 110 and are configured to sense temperature, measure electrical activity of tissue (e.g. IEGM signals), and/or perform other functions of an end effector sensor as understood by a person skilled in the art. The exposed electrically conductive strands can each have a thickness of approximately 25 micrometers or greater. The sum total of contact area of exposed region(s) of the exposed electrically conductive strands of the single electrode to tissue provides a cumulative contact area of about 0.004 square inches or greater to tissue, or about 0.006 square inches or greater to tissue. The exposed electrically conductive strands of the single electrode can be configured to delivery at least 900 volts and a current of about 20 amperes. The first strands 111 can be made of a material selected from nitinol, cobalt chromium, stainless steel, titanium. The second strands 112 can include nitinol, cobalt chromium, stainless steel, titanium.

As illustrated in the above examples, some or all of the strands 111, 112 can be wires that are electrically conductive. Portions of wires can be insulated or exposed, can function as an electrode, can provide an electrical connection to electrode(s) 170 coupled to the woven mesh 110, and/or can provide an electrical connection to sensor(s) 130 coupled to the woven mesh 110. The wires can be selectively shorted together to provide desired electrode and sensor functionality of the end effector 100.

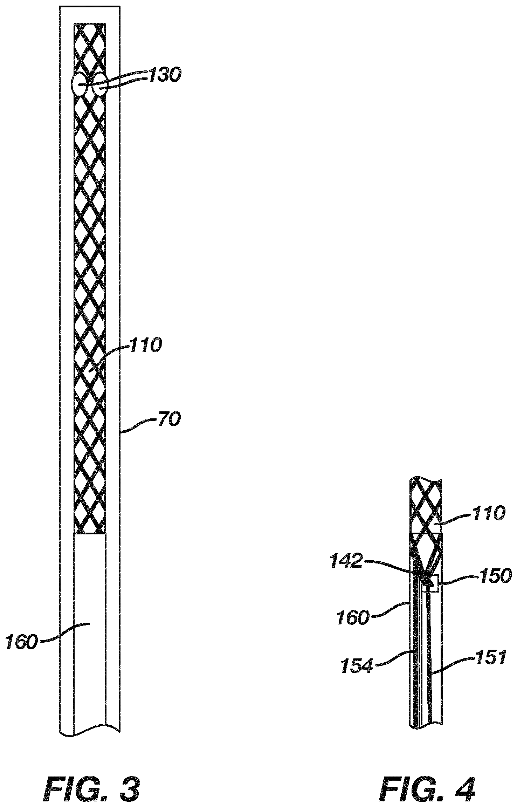

FIG. 3 is a schematic pictorial illustration showing a side view of the end effector 100 in a collapsed configuration, in accordance with the disclosed technology. The woven mesh 110 of the end effector 100 can be collapsed toward the longitudinal axis L-L so that the end effector 100 fits within a sheath catheter 70 for delivery through vasculature to a treatment site. The woven mesh 110 radially expands to the deployed configuration illustrated in FIGs. 1, 2A, and 2B after exiting a distal end of the sheath catheter 70. The end effector 100 can include an actuator 162 can be translated along the longitudinal axis L-L to facilitate collapse and expansion of the woven mesh 110 as illustrated in FIG. 2A, or the woven mesh 110 can be entirely self-expanding as illustrated in FIG. 2B. The woven mesh has a diameter approximately equal to a diameter R2 of the shaft 160 (FIGs. 2A and 2B) when in the collapsed, delivery configuration (FIG. 3). The woven mesh has a diameter R1 greater than the diameter R2 of the shaft 160 when in the expanded configuration (FIGs. 2A and 2B).

FIG. 4 is a schematic pictorial illustration showing a side view of the distal end of the shaft 160 joining the woven mesh 110 of the catheter 14 in a collapsed configuration. The shaft 160 is transparent for the sake of illustration. Proximal weave ends 142 of the woven mesh 110 are positioned within a distal portion of the shaft 160. A connector 150 mechanically and electrically couples a portion of the strands of the woven mesh to an electrical conductor 151. The shaft includes additional conductors 154 coupled to individual strands of the woven mesh 110. As disclosed in relation to FIGs 1, 2A, and 2B, strands 111, 112 (FIG. 2B) can have a number of electrical connection configurations to facilitate electrode(s) and sensor(s) of the end effector 100. The conductors 151, 154 provide connection from the strands 111, 112 to the PIU 30 (FIG. 1). As one example, the connector 150 and electrical conductor 151 can be electrically connected to a plurality of exposed electrically conductive strands that function as a single electrode of the end effector 100, and the multiple individual conductors 154 can be connected to insulated wires or strands to sensors and/or electrodes mounted to the woven mesh 110 that are electrically insulated from the strands joined by the connector 150.

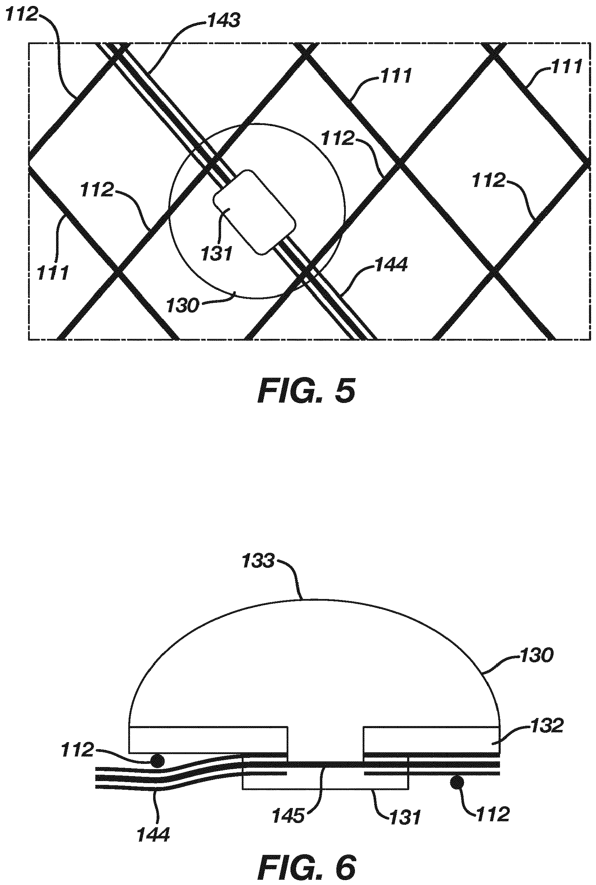

FIG. 5 is a schematic pictorial illustration showing a sensor 130 coupled to a woven mesh 110 and viewed from an internal volume of the braided mesh.

FIG. 6 is a schematic pictorial illustration showing a side view of the sensor 130 coupled to a woven mesh of FIG. 5.

Referring collectively to FIGs. 5 and 6, it is to be understood that an electrode 170 can be coupled in an identical manner as the sensor 130 so that FIGs. 5 and 6 show a sensor 130 or an electrode 170 coupled to the woven mesh 110. The woven mesh 110 can be configured as disclosed in relation to FIGs. 1 through 4. The woven mesh 110 includes first strands 111 and second strands 112. One of the first strands 111 is an insulated strand 143 (or an insulated conductive wire) electrically coupled to the sensor 130 (or electrode 170). The insulated strand 143 includes an insulator 144 (e.g. polymer sleeve or coating) and a conductor 145 (e.g. copper, nitinol, or other suitable metal). The sensor 130 has an exposed surface 133 on an outside of the woven mesh 110 configured to contact tissue. The sensor 130 includes a contact 131 configured to electrically and mechanically join the conductor 145 to the sensor 130 to provide electrical communication between the exposed surface 133 and the conductor 145 of the insulated strand 143. The insulation 144 of the insulated strand 143 electrically insulates the insulated strand 143 from other strands, including exposed electrically conductive strands, of the woven mesh 110. The sensor 130 (or electrode 170) can further include an electrically insulated backing 132 to electrically insulate the sensor 130 from any exposed portions of strands of the woven mesh 110 that may be in physical contact with the sensor 130. As shown in FIG. 5, in addition to being in physical and electrical contact with a first strand 143, the sensor 130 is also in physical contact with two second strands 112, which may include exposed electrically conductive portions. The insulated backing 132 can electrically insulate the sensor 130 (or electrode 170) from the second strands 112 and/or provide a surface to anchor the second strands 112 to the sensor 130 to provide a more robust connection of the sensor 130 (electrode 170) to the woven mesh 110.

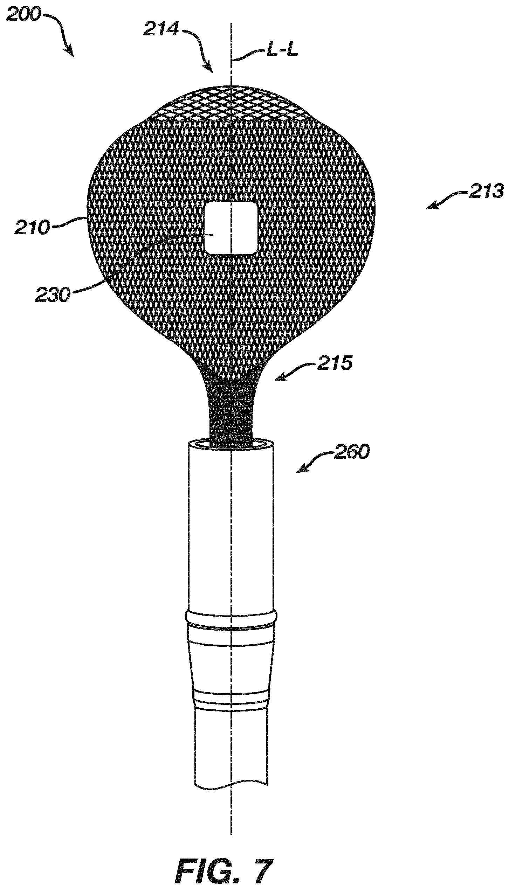

FIG. 7 is a schematic pictorial illustration showing a perspective view of a distal portion of another variation of the medical probe 14 illustrated in FIG. 1. The medical probe 14 includes an end effector 200 extending distally from the shaft 160 of the catheter 14. The end effector 200 can include a woven sheet 210 including a first plurality of wires 211 interwoven with a second plurality of wires 212 and formed into a teardrop shape 213 having a blunt distal end 214 and a tapered proximal end 215. At least a portion of the second plurality of wires 212 are configured to deliver ablation energy to tissue. A sensor or electrode 230 is attached to at least one of the first plurality of wires 211.

In some examples, the first plurality of wires 211 can be non-overlapping with each other, the second plurality of wires 212 can be non-overlapping with each other, and at least a portion of the first plurality of wires 211 can overlap with at least a portion of the second plurality of wires 212.

The end effector 200 can include a shaft 160 extending along a longitudinal axis L-L such that the woven mesh 210 extends distally from a distal end of the shaft 160. The woven mesh 210 can be configured to radially expand from a collapsed delivery configuration (similar to FIG. 3) to an expanded deployed configuration.

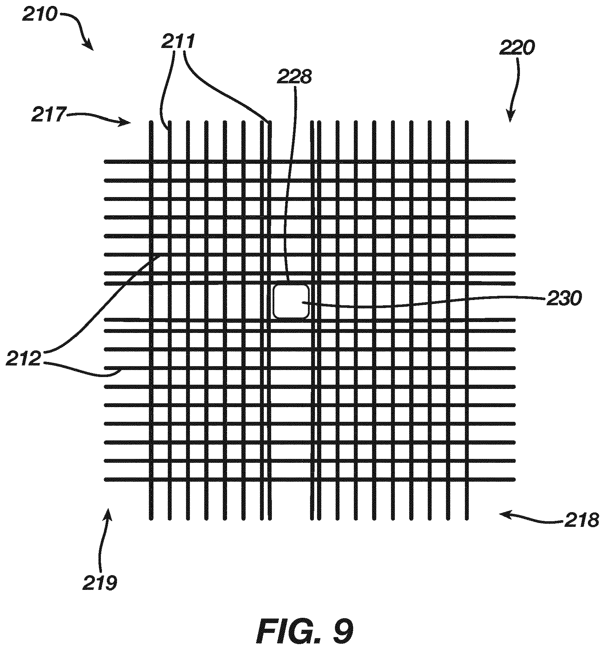

The end effector 200 can be made of a wire mesh 210, with the plurality of electrically conductive strands 211 (FIG. 9) having expose portions configured to contact tissue. The end effector 200 can include one or more electrodes 230 electrically coupled to one or more strands of the plurality of electrically conductive strands 211 and configured to deliver ablation energy to tissue. The plurality of electrically conductive strands are electrically coupled to each other to form an ablation electrode. Additionally, or alternatively, the end effector 200 can include one or more sensors 230 electrically coupled to one or more strands of the plurality of electrically conductive strands 211 configured to determine deformation of the woven mesh, sense temperature, measure electrical activity of tissue (e.g. IEGM signals), and/or perform other functions of an end effector sensor as understood by a person skilled in the art.

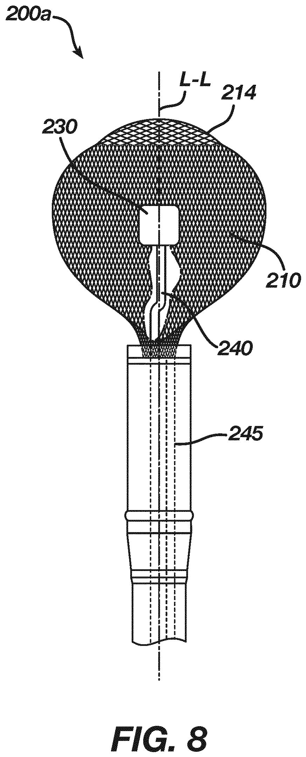

FIG. 8 is a schematic pictorial illustration showing a side view of an atraumatic end effector 200a that is a variation of the end effector 200 illustrated in FIG. 7. The end effector 200a can be made of a wire mesh 210 as described in relation to FIG. 7, with the end effector 200a including a wire 240 electrically coupled to at least one of the plurality of sensors or electrodes 230. The wire 240 can be electrically isolated from at least a portion of the plurality of conductive strands 211 (FIG. 9). The wire 240 can be woven in the woven mesh 210. The medical probe 14 can include a second wire 245 electrically connected to the wire mesh 210.

Examples of materials ideally suited for forming electrodes include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

FIG. 9 is a schematic pictorial illustration showing a top view of a woven mesh 210. In some examples, the woven mesh can include a woven sheet 210 having an electrode gap 228 with the sensor or electrode 230 disposed in the electrode gap 228. The electrode gap 228 can be sized to accommodate the sensor or electrode 230. The woven mesh 210 includes a first plurality of wires 211 in a first direction and a second plurality of wires 212 in a second direction. Each of the first plurality of wires 211 have a first end 217 and a second end 218. Each of the second plurality of wires 212 have a first end 219 and a second end 220.

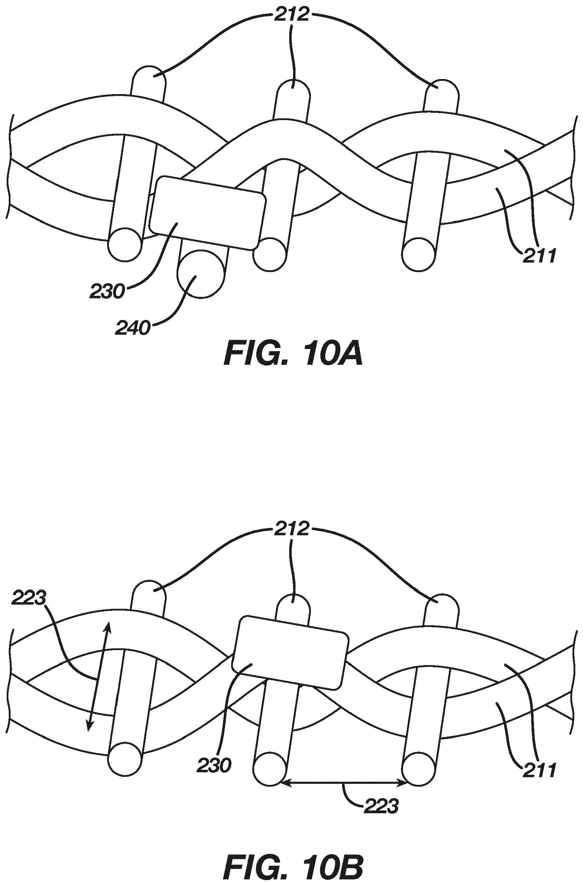

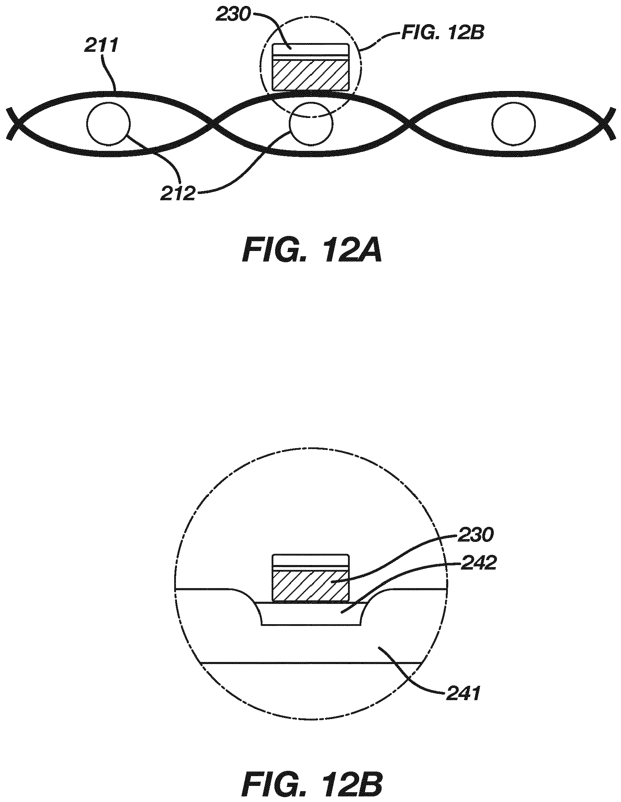

In some examples, a plurality of welds can hold the first plurality of wires 211 to the second plurality of wires 212. The plurality of welds can be placed to maintain a distance 223 of about 0.2 mm between each of the first plurality of wires 211 and two parallel adjacent wires and shown in FIG. 10B. As shown in FIG. 10A, an insulated conductive wire 240 can be physically coupled to the woven sheet 210 and in electrical communication with the sensor or electrode 230. Alternatively or additionally, as shown in FIG. 10B at least one of the first plurality of wires 211 can include an outer insulating layer 241 and an uncovered portion 242, the sensor or electrode 230 attached to and in electrical communication with the uncovered portion 242. This is shown in more detail in FIG. 12A and the magnified view FIG. 12B.

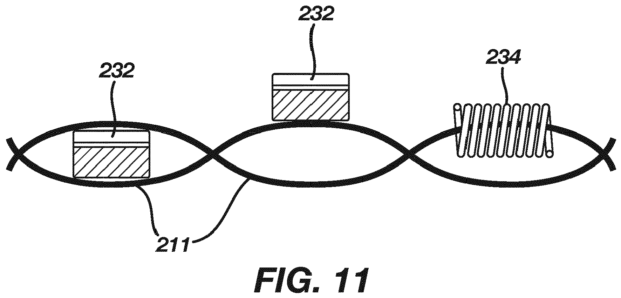

As shown in FIG. 11, at least one of the first plurality of wires 211 can be woven over the sensor or electrode 232, at least one of the first plurality of wires 211 can be woven under the sensor or electrode 232, at least one of the second plurality of wires 212 can be woven over the sensor or electrode 232, and at least one of the second plurality of wires 212 can be woven under the sensor or electrode 232 such that the sensor or electrode 232 is woven into the woven sheet 210. In some examples, at least one of the first plurality of wires 211 can be woven over the sensor or electrode 232 and electronically coupled to the sensor or electrode 230. The placement of the woven wires 211, 212 in relation to the sensor 232 can be such that the wires reduce the likelihood that the sensors 232 will snag, for example on tissue or insertion catheters 70, while still allowing contact between the sensor 232 and tissue. The woven mesh can also include a sensor or electrode 233 which is not woven into the mesh but rather attached to the outside as described herein.

The end effector can include a plurality of sensors 230, the sensor 230 being one of the plurality of the sensors 230. The plurality of sensors 230 can be configured to determine deformation of the woven mesh 210, sense temperature, and/or measure electrical activity of tissue. The second plurality of wires 212 can be configured to deliver electrical pulses having a peak voltage of at least 900 volts V and have a current rating of at least 20 amperes. The first plurality of wires 211 can be made of nitinol, cobalt chromium, stainless steel, or titanium. The second plurality of wires 212 can include nitinol, cobalt chromium, stainless steel, or titanium.

In some examples, the basket can include a helical electrode 234 wrapped helically around at least one of the first plurality of wires 211. The helical electrode 234 can be coupled to any of the first plurality of wires 211 as previously described. Helical electrode 234 can have the advantage of flexing with the wire or plurality of wires to which it is attached.

In some examples, the second plurality of wires 212 can include a plurality of exposed portions and configured to contact tissue to apply the ablation energy to tissue. The plurality of exposed portions can be configured to provide a cumulative contact area to tissue of about 0.004 square inches. In other examples, the plurality of exposed portions configured to provide a cumulative contact area to tissue of about 0.006 square inches.

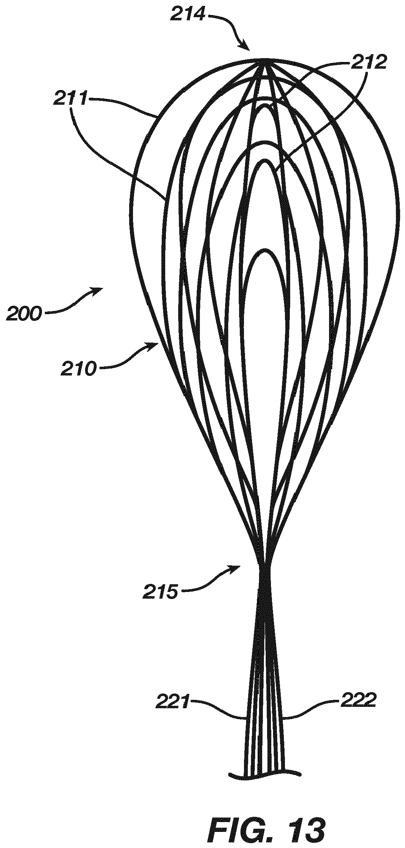

FIG. 13 is a schematic pictorial illustration showing a perspective view of a basket of an atraumatic medical probe, in accordance with the disclosed technology; each of the first plurality of wires 211 having a first end 217 and a second end 218 (FIG. 9) gathered proximate the tapered proximal end 215, and each of the second plurality of wires 212 having a first end 219 and a second end 220 (FIG. 9) gathered proximate the tapered proximal end 215. In some examples, the gathered first end 217 and second end 218 (FIG. 9) of each of the first plurality of wires 211 joined to a first main wire 221, and the gathered first end 219 and second end 220 (FIG. 9) of each of the second plurality of wires 212 joined to a second main wire 222.

Referring collectively to FIG. 7 through 13, some examples of the end effector of the medical probe described previously can be a basket including a woven sheet 210 include a first plurality of wires 211 oriented in a first direction, each of the first plurality of wires having a first end 217 and a second end 218 (FIG. 9) and a second plurality of wires 212 oriented in a second direction parallel to the first direction, the first plurality of wires 211 and the second plurality of wires 212 woven together in a plain weave pattern and formed into a teardrop shape 213 (FIG. 7) having a blunt distal end 214 (FIG. 7 and FIG. 13) and a tapered proximal end 215 (FIG. 13), each of the second plurality of wires 212 having a first end 219 and a second end 220 (FIG. 9), the second plurality of wires 212 interwoven with the first plurality of wires 211, the first end 217 and the second end 218 of at least one of the first plurality of wires 211 joined to another of the first plurality of wires 211, and the first end 217 and the second end 218 of at least one of the second plurality of wires 219 joined to another of the second plurality of wires 212 (FIG. 13).

The basket can further include a first main wire 221 and a second main wire 222 (FIG. 13). The first plurality of wires 211 can be connected to the first main wire 221 and the second plurality of wires 212 can be connected to the second main wire 221. The first plurality of wires 211 can be insulated by an insulating layer 241 and having a plurality of openings 242 in the insulating layer 241, with the electrode 230 coupled to the openings 242 (FIG. 12B).

The second plurality of wires 241 can include a plurality of exposed portions and can be configured to apply energy capable of inducing irreversible electroporation in a target tissue (FIG. 12B).

The insulative layer 241 and other insulating components described herein can be made from a biocompatible, electrically insulative material such as polyamide-polyether (Pebax^{®}) copolymers, polyethylene terephthalate (PET), urethanes, polyimide, parylene, fluoropolymers (such as PTFE, ePTFE, ETFE, FEP, PFA), silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets may also include one or more additives or fillers, such as, for example, polytetrafluoroethylene (PTFE), barium sulfate (BaSO₄), bismuth oxide (Bi₂O₃), bismuth oxychloride (BiOCl), boron nitride, silicon nitride, silicon carbide, aluminum oxide, aluminum nitride, zinc oxide, and the like.

FIG. 14 is an illustration of a variation of the catheter 14 (FIG. 1) having an alternatively shaped end effector 300. The end effector 300 includes a woven mesh 310 with distal weave ends joined by a band 314. The distal weave ends are outside of the interior volume defined by the woven mesh 310. The woven mesh 310 is inverted at the distal end to provide an atraumatic distal surface to the woven mesh 310, the band 314 being sunken into the distal end of the woven mesh to be inhibited from impinging tissue. The end effector 300 can be modified to include sensors, strands, electrodes, etc. as understood by a person skilled in the art according to the teachings herein.

FIG. 15 is an illustration of another variation of the catheter 14 (FIG. 1) having an alternatively shaped end effector 400 with a woven mesh 410 that is continuous distal of the shaft, meaning weave ends of the woven mesh 410 are within the shaft 160. The weave pattern of the woven mesh 410 is continuous across the distal end 414 of the woven mesh 410. The end effector 400 can be modified to include sensors, strands, electrodes, etc. as understood by a person skilled in the art according to the teachings herein.

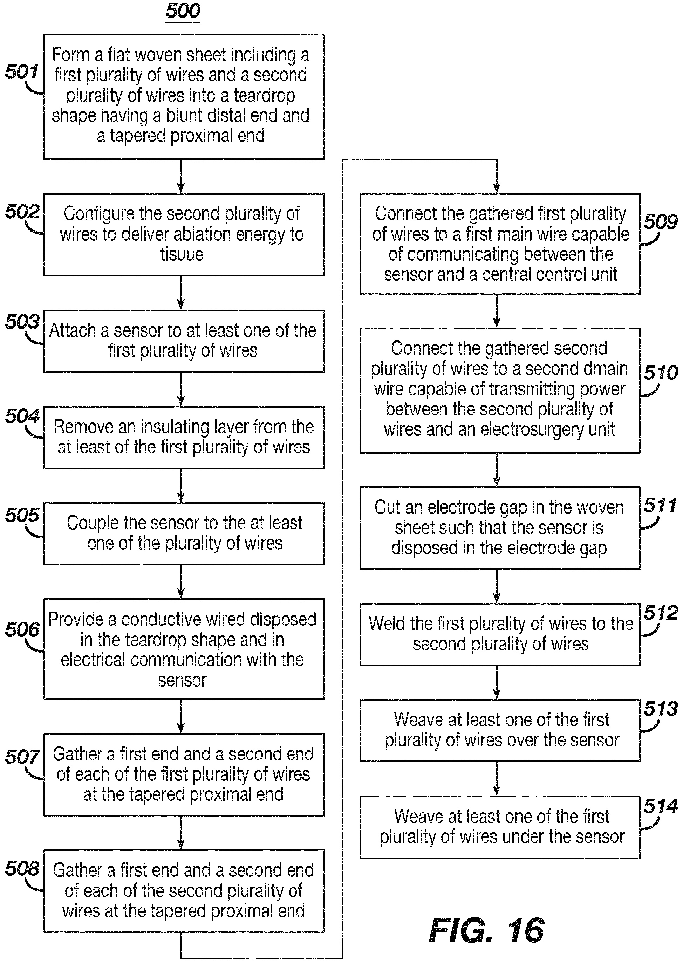

FIG. 16 is a flowchart illustrating a method of making an atraumatic medical probe (e.g., atraumatic medical probe 14), in accordance with an embodiment of the present invention. The method 500 can include forming (step 501) a flat woven sheet including a first plurality of wires and a second plurality of wires into a teardrop shape having a blunt distal end and a tapered proximal end, configuring (step 502) the second plurality of wires to deliver ablation energy to tissue, and attaching (step 503) a sensor to at least one of the first plurality of wires.

In some examples, the method 500 can include removing (step 504) an insulating layer from the at least one of the first plurality of wires and electrically coupling (step 505) the sensor to the at least one of the first plurality of wires.

In some examples, the method 500 can include providing (step 506) a conductive wire disposed in the teardrop shape and in electrical communication with the sensor.

In some examples, the method 500 can include gathering (step 507) a first end and a second end of each of the first plurality of wires at the tapered proximal end, gathering (step 508) a first end and a second end of each of the second plurality of wires at the tapered proximal end connecting (step 509) the gathered first plurality of wires to a first main wire capable communicating between the sensor and a central control unit, and connecting (step 510) the gathered second plurality of wires to a second main wire capable transmitting power between the second plurality of wires and an electrosurgery unit.

In some examples, the method 500 can include cutting (step 511) an electrode gap in the woven sheet, the sensor disposed in the electrode gap and the electrode gap sized to accommodate the sensor.

In some examples, the method 500 can include welding (step 512) the first plurality of wires to the second plurality of wires, the plurality of welds placed to maintain a distance of about 0.2 mm between each of the first plurality of wires and two parallel adjacent wires.

In some examples, the method 500 can include weaving (step 513) at least one of the first plurality of wires over the sensor, and weaving (step 514) at least one of the first plurality of wires woven under the sensor. The method 500 can further include weaving at least one of the second plurality of wires woven over the sensor, and weaving at least one of the second plurality of wires woven under the sensor such that the sensor is woven into the woven sheet.

As will be appreciated, the method 500 just described can be varied in accordance with the various elements and implementations described herein. That is, methods in accordance with the disclosed technology can include all or some of the steps described above and/or can include additional steps not expressly disclosed above. Further, methods in accordance with the disclosed technology can include some, but not all, of a particular step described above. Further still, various methods described herein can be combined in full or in part. That is, methods in accordance with the disclosed technology can include at least some elements or steps of a first method and at least some elements or steps of a second method.

The disclosed technology described herein can be further understood according to the following clauses:
Clause 1: An end effector in a medical catheter comprising a woven mesh extending along a longitudinal axis from a proximal portion to a distal portion, the woven mesh defining a generally spheroidal shape having a plurality of electrodes disposed about the longitudinal axis and adjacent to an equator of the generally spheroidal shape, the proximal portion configured to be attached to a shaft; and the distal portion configured to be invaginated and coupled to an actuator extending along the longitudinal axis.
Clause 2: The end effector of clause 1, wherein the woven mesh comprises a plurality of electrically insulated strands and a plurality of exposed electrically conductive strands, the plurality of electrically insulated strands overlapping the plurality of exposed electrically conductive strands.
Clause 3: The end effector of any one of clauses 1-2, wherein the plurality of exposed electrically conductive strands are configured to provide a cumulative contact area of about 0.006 square inches or greater to tissue such that the cumulative contact area is a sum total of contact area of exposed regions of the plurality of exposed electrically conductive strands to tissue.
Clause 4: The end effector of any one of clauses 1-3, wherein the end effector is configured to deliver an ablation energy to tissue in the form of irreversible electroporation pulses.
Clause 5: The end effector of any one of clauses 1-4, wherein the end effector is configured to deliver an ablation energy to tissue in the form of radio frequency thermal ablation.
Clause 6: The end effector of any one of clauses 1-5 wherein the woven mesh is formed from a woven sheet.
Clause 7: The end effector of any one of clauses 1-5, wherein the woven mesh is formed from a braided tube.
Clause 8: An electrode comprising a woven mesh comprising a collapsed configuration and a radially expanded deployed configuration, the woven mesh comprising a plurality of exposed electrically conductive strands configured to deliver ablation energy to tissue, the plurality of exposed electrically conductive strands provides a cumulative contact area of about 0.004 square inches or greater to tissue such that the cumulative contact area is a sum total of contact area of exposed regions of the plurality of exposed electrically conductive strands to tissue to deliver at least 900 volts and a current of about 20 amperes.
Clause 9: The electrode of clause 8, wherein the plurality of exposed electrically conductive strands comprises nitinol.
Clause 10: The electrode of clause 8 or 9, wherein the woven mesh is configured to self-expand to the deployed configuration.
Clause 11: The electrode of any one of clauses 8-9, wherein strands of the plurality of exposed electrically conductive strands each comprise a thickness of approximately 25 micrometers or greater.
Clause 12: The electrode of any one of clauses 8-10, wherein the plurality of exposed electrically conductive strands are non-overlapping with each other.
Clause 13: The electrode of clause 12, wherein the woven mesh comprises a plurality of electrically insulated strands overlapping with the plurality of exposed electrically conductive strands.
Clause 14: The electrode of any one of clauses 8-13, wherein the plurality of exposed electrically conductive strands are configured to provide a cumulative contact area of about 0.006 square inches or greater to tissue such that the cumulative contact area is a sum total of contact area of exposed regions of the plurality of exposed electrically conductive strands to tissue.
Clause 15: The electrode of any one of clauses 8-14, wherein the electrode is configured to deliver the ablation energy to tissue in the form of irreversible electroporation pulses.
Clause 16: The electrode of any one of clauses 8-15, wherein the electrode is configured to deliver the ablation energy to tissue in the form of radio frequency thermal ablation.
Clause 17: The electrode of any one of clauses 8-16, wherein the woven mesh is formed from a woven sheet.
Clause 18: The electrode of any one of clauses 8-16, wherein the woven mesh is formed from a braided tube.
Clause 19: A medical probe comprising a woven mesh comprising a plurality of electrically conductive strands configured to deliver ablation energy to tissue; and a plurality of sensors coupled to the woven mesh.
Clause 20: The medical probe of clause 19, wherein the plurality of sensors are configured to determine deformation of the woven mesh, sense temperature, and/or measure electrical activity of tissue.
Clause 21: The medical probe of clause 19 or 20, further comprising a wire electrically coupled to at least one of the plurality of sensors, the wire being electrically isolated from at least a portion of the plurality of conductive strands.
Clause 22: The medical probe of clause 21, wherein the wire is woven in the woven mesh.
Clause 23: The medical probe of any one of clauses 19-22, further comprising a shaft extending along a longitudinal axis such that the woven mesh extends distally from a distal end of the shaft.
Clause 24: The medical probe of clause 23, wherein the woven mesh is configured to radially expand from a collapsed delivery configuration to an expanded deployed configuration, and wherein the woven mesh comprises a diameter approximately equal to a diameter of the shaft when in the delivery configuration and a diameter greater than the shaft when in the expanded configuration.
Clause 25: The medical probe of clause 23 or 24, wherein the woven mesh comprises a plurality of weave ends disposed within a distal portion of the shaft.
Clause 26: The medical probe of any one of clauses 23-25, wherein the woven mesh comprises a contiguous weave distal of the distal end of the shaft.
Clause 27: The medical probe of any one of clauses 23-25, wherein the woven mesh comprises a plurality of weave ends disposed approximate a distal end of the medical probe.
Clause 28: The medical probe of any one of clauses 23-25, wherein the woven mesh defines an internal volume, and wherein a plurality of weave ends are disposed within the internal volume.
Clause 29: The medical probe of any one of clauses 19-28, wherein the plurality of electrically conductive strands comprises expose portions configured to contact tissue.
Clause 30: The medical probe of any one of clauses 19-29, further comprising one or more electrodes electrically coupled to one or more strands of the plurality of electrically conductive strands and configured to deliver ablation energy to tissue.
Clause 31: The medical probe of any one of clauses 19-29, wherein the plurality of electrically conductive strands are electrically coupled to each other to form an ablation electrode.
Clause 32: An atraumatic medical probe comprising a woven sheet comprising a first plurality of wires interwoven with a second plurality of wires and formed into a teardrop shape comprising a blunt distal end and a tapered proximal end, at least a portion of the second plurality of wires being configured to deliver ablation energy to tissue; and a sensor attached to at least one of the first plurality of wires.
Clause 33: The medical probe of clause 32, wherein the first plurality of wires are non-overlapping with each other, the second plurality of wires are non-overlapping with each other, and at least a portion of the first plurality of wires overlap with at least a portion of the second plurality of wires.
Clause 34: The medical probe of clause 32 or 33, further comprising: an insulated conductive wire physically coupled to the woven sheet and in electrical communication with the sensor.
Clause 35: The medical probe of any one of clauses 32-34, at least one of the first plurality of wires comprising an outer insulating layer and an uncovered portion, the sensor attached to and in electrical communication with the uncovered portion.
Clause 36: The medical probe of any one of clauses 32-35, the second plurality of wires comprising a plurality of exposed portions and configured to contact tissue to apply the ablation energy to tissue.
Clause 37: The medical probe of clause 36, the plurality of exposed portions configured to provide a cumulative contact area to tissue of about 0.004 square inches.
Clause 38: The medical probe of clause 36, the plurality of exposed portions configured to provide a cumulative contact area to tissue of about 0.006 square inches.
Clause 39: The medical probe according to any of clauses 32-38, each of the first plurality of wires having a first end and a second end gathered proximate the tapered proximal end, and each of the second plurality of wires having a first end and a second end gathered proximate the tapered proximal end.
Clause 40: The medical probe of clause 39, the gathered first end and second end of each of the first plurality of wires joined to a first main wire, and the gathered first end and second end of each of the second plurality of wires joined to a second main wire.
Clause 41: The medical probe according to any of clauses 32-40, the woven sheet further comprising an electrode gap, the sensor disposed in the electrode gap and the electrode gap sized to accommodate the sensor.
Clause 42: The medical probe according to any of clauses 32-41, further comprising a plurality of welds holding the first plurality of wires to the second plurality of wires.
Clause 43: The medical probe of clause 42, the plurality of welds placed to maintain a distance of about 0.2 mm between each of the first plurality of wires and two parallel adjacent wires.
Clause 44: The medical probe according to any of clauses 32-43, at least one of the first plurality of wires woven over the sensor, at least one of the first plurality of wires woven under the sensor, at least one of the second plurality of wires woven over the sensor, and at least one of the second plurality of wires woven under the sensor such that the sensor is woven into the woven sheet.
Clause 45: The medical probe of clause 44, the at least one of the first plurality of wires woven over the sensor and electronically coupled to the sensor.
Clause 46: The medical probe according to any of clauses 32-45 further comprising a plurality of sensors, the sensor being one of the plurality of the sensors.
Clause 47: The medical probe according to any of clauses 32-46, wherein the second plurality of wires is configured to deliver electrical pulses having a peak voltage of at least 900 volts V.
Clause 48: The medical probe according to any of clauses 32-47, wherein the first plurality of wires comprises a material selected from a group consisting of nitinol, cobalt chromium, stainless steel, titanium, and wherein the second plurality of wires comprises a material selected from a group consisting of nitinol, cobalt chromium, stainless steel, titanium.
Clause 49: A method of making an atraumatic medical probe, the method comprising: forming a flat woven sheet comprising a first plurality of wires and a second plurality of wires into a teardrop shape comprising a blunt distal end and a tapered proximal end; configuring the second plurality of wires to deliver ablation energy to tissue; and attaching a sensor to at least one of the first plurality of wires.
Clause 50: The method of clause 49, further comprising: removing an insulating layer from the at least one of the first plurality of wires; and electrically coupling the sensor to the at least one of the first plurality of wires.
Clause 51: The method of clause 49, further comprising: providing a conductive wire disposed in the teardrop shape and in electrical communication with the sensor.
Clause 52: The method according to any of clauses 49-51, further comprising: gathering a first end and a second end of each of the first plurality of wires at the tapered proximal end; gathering a first end and a second end of each of the second plurality of wires at the tapered proximal end; connecting the gathered first plurality of wires to a first main wire capable communicating between the sensor and a central control unit; and connecting the gathered second plurality of wires to a second main wire capable transmitting power between the second plurality of wires and an electrosurgery unit.
Clause 53: The method according to any of clauses 49-52, further comprising cutting an electrode gap in the woven sheet, the sensor disposed in the electrode gap and the electrode gap sized to accommodate the sensor.
Clause 54: The method according to any of clauses 49-53 further comprising welding the first plurality of wires to the second plurality of wires, the plurality of welds placed to maintain a distance of about 0.2 mm between each of the first plurality of wires and two parallel adjacent wires.
Clause 55: The method according to any of clauses 49-54 further comprising: weaving at least one of the first plurality of wires over the sensor; weaving at least one of the first plurality of wires woven under the sensor; weaving at least one of the second plurality of wires woven over the sensor; and weaving at least one of the second plurality of wires woven under the sensor such that the sensor is woven into the woven sheet.
Clause 56: The method according to any of clauses 49-55, wherein the first plurality of wires comprises a material selected from a group consisting of nitinol, cobalt chromium, stainless steel, titanium, and wherein the second plurality of wires comprises a material selected from a group consisting of nitinol, cobalt chromium, stainless steel, titanium.
Clause 57: A basket for a medical probe comprising: a woven sheet comprising: a first plurality of wires oriented in a first direction, each of the first plurality of wires having a first end and a second end; and a second plurality of wires oriented in a second direction parallel to the first direction, the first plurality of wires and the second plurality of wires woven together in a plain weave pattern and formed into a teardrop shape comprising a blunt distal end and a tapered proximal end, each of the second plurality of wires having a first end and a second end, the second plurality of wires interwoven with the first plurality of wires, the first end and the second end of at least one of the first plurality of wires joined to another of the first plurality of wires, and the first end and the second end of at least one of the second plurality of wires joined to another of the second plurality of wires.
Clause 58: The basket of clause 57, further comprising: a first main wire; and a second main wire, the first plurality of wires connected to the first main wire and the second plurality of wires connected to the second main wire.
Clause 59: The basket according to any of clauses 57-58, further comprising: a helical electrode wrapped helically around at least one of the first plurality of wires.
Clause 60: The basket according to any of clauses 57-59, the first plurality of wires being insulated by an insulating layer and comprising a plurality of openings in the insulating layer.
Clause 61: The basket according to any of clauses 57-60, the second plurality of wires comprising a plurality of exposed portions and configured to apply energy capable of inducing irreversible electroporation in a target tissue.
Clause 62: The basket according to any of clauses 57-61, the woven sheet further comprising an electrode gap sized to accommodate an electrode.
Clause 63: The basket according to any of clauses 57-62, further comprising a plurality of welds holding the first plurality of wires to the second plurality of wires.
Clause 64: The basket of clause 63, the plurality of welds placed to maintain a distance of about 0.2 mm between each of the first plurality of wires and two parallel adjacent wires.
Clause 65: The basket according to any of clauses 57-64, wherein the first plurality of wires comprises a material selected from a group consisting of nitinol, cobalt chromium, stainless steel, titanium, and wherein the second plurality of wires comprises a material selected from a group consisting of nitinol, cobalt chromium, stainless steel, titanium.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An end effector in a medical catheter comprising;
a woven mesh extending along a longitudinal axis from a proximal portion to a distal portion, the woven mesh defining a generally spheroidal shape having a plurality of electrodes disposed about the longitudinal axis and adjacent to an equator of the generally spheroidal shape;
the proximal portion configured to be attached to a shaft; and
the distal portion configured to be invaginated and coupled to an actuator extending along the longitudinal axis.

2. The end effector of claim 1, wherein the woven mesh comprises a plurality of electrically insulated strands and a plurality of exposed electrically conductive strands, the plurality of electrically insulated strands overlapping the plurality of exposed electrically conductive strands, optionally wherein the plurality of exposed electrically conductive strands are configured to provide a cumulative contact area of about 0.006 square inches or greater to tissue such that the cumulative contact area is a sum total of contact area of exposed regions of the plurality of exposed electrically conductive strands to tissue.

3. The end effector of claim 1, wherein the end effector is configured to deliver an ablation energy to tissue in the form of irreversible electroporation pulses, or radio frequency thermal ablation.

4. The end effector of claim 1 wherein the woven mesh is formed from a woven sheet.

5. An atraumatic medical probe comprising:
a woven sheet comprising a first plurality of wires interwoven with a second plurality of wires and formed into a teardrop shape comprising a blunt distal end and a tapered proximal end, at least a portion of the second plurality of wires being configured to deliver ablation energy to tissue; and
a sensor attached to at least one of the first plurality of wires.

6. The medical probe of claim 5, wherein the first plurality of wires are non-overlapping with each other, the second plurality of wires are non-overlapping with each other, and at least a portion of the first plurality of wires overlap with at least a portion of the second plurality of wires.

7. The medical probe of claim 5, further comprising:
an insulated conductive wire physically coupled to the woven sheet and in electrical communication with the sensor.

8. The medical probe of claim 5, at least one of the first plurality of wires comprising an outer insulating layer and an uncovered portion, the sensor attached to and in electrical communication with the uncovered portion.

9. The medical probe of claim 5, the second plurality of wires comprising a plurality of exposed portions and configured to contact tissue to apply the ablation energy to tissue.

10. The medical probe of claim 5, each of the first plurality of wires having a gathered first end and a second end gathered proximate the tapered proximal end, and each of the second plurality of wires having a first end and a second end gathered proximate the tapered proximal end, optionally the gathered first end and second end of each of the first plurality of wires joined to a first main wire, and the gathered first end and second end of each of the second plurality of wires joined to a second main wire.

11. The medical probe of claim 5, the woven sheet further comprising an electrode gap, the sensor disposed in the electrode gap and the electrode gap sized to accommodate the sensor.

12. The medical probe of claim 5, at least one of the first plurality of wires woven over the sensor and electronically coupled to the sensor, at least one of the first plurality of wires woven under the sensor, at least one of the second plurality of wires woven over the sensor, and at least one of the second plurality of wires woven under the sensor such that the sensor is woven into the woven sheet.

13. A method of making an atraumatic medical probe, the method comprising:
forming a flat woven sheet comprising a first plurality of wires and a second plurality of wires into a teardrop shape comprising a blunt distal end and a tapered proximal end;
configuring the second plurality of wires to deliver ablation energy to tissue; and
attaching a sensor to at least one of the first plurality of wires.

14. The method of claim 13, further comprising:
removing an insulating layer from the at least one of the first plurality of wires;
electrically coupling the sensor to the at least one of the first plurality of wires; and
providing a conductive wire disposed in the teardrop shape and in electrical communication with the sensor.

15. The method of claim 14, further comprising:
gathering a first end and a second end of each of the first plurality of wires at the tapered proximal end;
gathering a first end and a second end of each of the second plurality of wires at the tapered proximal end;
connecting the gathered first plurality of wires to a first main wire capable communicating between the sensor and a central control unit; and
connecting the gathered second plurality of wires to a second main wire capable transmitting power between the second plurality of wires and an electrosurgery unit;
optionally further comprising cutting an electrode gap in the woven sheet, the sensor disposed in the electrode gap and the electrode gap sized to accommodate the sensor;
further optionally further comprising:
weaving at least one of the first plurality of wires over the sensor;
weaving at least one of the first plurality of wires woven under the sensor;
weaving at least one of the second plurality of wires woven over the sensor; and
weaving at least one of the second plurality of wires woven under the sensor such that the sensor is woven into the woven sheet.
